# EUROPEAN PATENT SPECIFICATION

(11) **EP 1 861 509 B1**
(45) Date of publication and mention of the grant of the patent: **09.09.2015**
(21) Application number: 05725638.0
(22) Date of filing: 14.03.2005
(51) Int. Cl.: C12Q 1/00, G01N 33/48, G01N 33/53, G01N 33/574

(54) **A METHOD FOR PREDICTING PROGRESSION FREE AND OVERALL SURVIVAL AT EACH FOLLOW-UP TIME POINT DURING THERAPY OF METASTATIC BREAST CANCER PATIENTS USING CIRCULATING TUMOR CELLS**
VERFAHREN ZUR VORHERSAGE DES ÜBERLEBENS INSGESAMT UND OHNE KRANKHEITSFORTSCHREITUNG ZU JEDEM NACHSORGEZEITPUNKT BEI DER THERAPIE VON METASTATISCHEN BRUSTKREBSPATIENTEN UNTER VERWENDUNG ZIRKULIERENDER TUMORZELLEN
MÉTHODE DE PRÉDICTION DE SURVIE SANS PROGRESSION ET DE SURVIE GLOBALE, À CHAQUE INSTANT DU SUIVI, AU COURS DU TRAITEMENT DE PATIENTES ATTEINTES DU CANCER DU SEIN MÉTASTATIQUE, UTILISANT DES CELLULES TUMORALES CIRCULANTES

(43) Date of publication of application: 05.12.2007
(73) Proprietor: Janssen Diagnostics, LLC, North Raritan, NJ 08869 (US)
(72) Inventor: ALLARD, Jeffrey, W., North Wales, PA 19454 (US); DOYLE, Gerald, V., Radnor, PA 19087 (US); HAYES, Daniel, F. Comprehensive Cancer Center, Ann Arbor, MI 48109-0942 (US); MILLER, Michael, Craig, Quakertown, PA 18951 (US); TERSTAPPEN, Leon, W., M., M., Huntingdon Valley, PA 19006 (US)
(74) Representative: Hanson, William Bennett
(86) International application number: PCT/US2005/008602
(87) International publication number: WO 2006/104474

(56) References cited:
- WO-A1-99/41613
- US-A- 5 993 388
- US-A1- 2002 098 535
- CRISTOFANILLI MASSIMO ET AL: "Circulating tumor cells: a novel prognostic factor for newly diagnosed metastatic breast cancer." JOURNAL OF CLINICAL ONCOLOGY : OFFICIAL JOURNAL OF THE AMERICAN SOCIETY OF CLINICAL ONCOLOGY 1 MAR 2005, vol. 23, no. 7, 1 March 2005 (2005-03-01), pages 1420-1430, XP002570579 ISSN: 0732-183X
- CRISTOFANILLI MASSIMO ET AL: "Circulating tumor cells, disease progression, and survival in metastatic breast cancer" NEW ENGLAND JOURNAL OF MEDICINE, vol. 351, no. 8, 19 August 2004 (2004-08-19), pages 781-791, XP002570580 ISSN: 0028-4793

## Description

### Background

### • Field of the Invention

The invention relates generally to cancer prognosis and survival in metastatic cancer patients, based on the presence of morphologically intact circulating cancer cells (CTC) in blood. More specifically, diagnostic methods, reagents and apparatus are described that correlate the presence of circulating cancer cells in 7.5 ml of blood of metastatic breast cancer patients with time to disease progression and survivability. Circulating tumor cells are determined by highly sensitive methodologies capable of isolating and imaging 1 or 2 cancer cells in approximately 5 to 50 ml of peripheral blood, the level of the tumor cell number and an increase in tumor cell number during treatment are correlated to the time to progression, time to death and response to therapy.

### • Background Art

Despite efforts to improve treatment and management of cancer patients, survival in cancer patients has not improved over the past two decades for many cancer types. Accordingly, most cancer patients are not killed by their primary tumor, but they succumb instead to metastases: multiple widespread tumor colonies established by malignant cells that detach themselves from the original tumor and travel through the body, often to distant sites. The most successful therapeutic strategy in cancer is early detection and surgical removal of the tumor while still organ confined. Early detection of cancer has proven feasible for some cancers, particularly where appropriate diagnostic tests exist such as PAP smears in cervical cancer, mammography in breast cancer, and serum prostate specific antigen (PSA) in prostate cancer. However, many cancers detected at early stages have established micrometastases prior to surgical resection. Thus, early and accurate determination of the cancer's malignant potential is important for selection of proper therapy.

Optimal therapy will be based on a combination of diagnostic and prognostic information. An accurate and reproducible diagnostic test is needed to provide specific information regarding the metastatic nature of a particular cancer, together with a prognostic assessment that will provide specific information regarding survival.

A properly designed prognostic test will give physicians information about risk and likelihood of survival, which in turn gives the patient the benefit of not having to endure unnecessary treatment. Patient morale would also be boosted from the knowledge that a selected therapy will be effective based on a prognostic test. The cost savings associated with such a test could be significant as the physician would be provided with a rationale for replacing ineffective therapies. A properly developed diagnostic and prognostic data bank in the treatment and detection of metastatic cancer focusing on survival obviously would provide an enormous benefit to medicine (US 6,063,586).

If a primary tumor is detected early enough, it can often be eliminated by surgery, radiation, or chemotherapy or some combination of those treatments. Unfortunately, the metastatic colonies are difficult to detect and eliminate and it is often impossible to treat all of them successfully. Therefore from a clinical point of view, metastasis can be considered the conclusive event in the natural progression of cancer. Moreover, the ability to metastasize is a property that uniquely characterizes a malignant tumor.

### Soluble Tumor Antigen:

Based on the complexity of cancer and cancer metastasis and the frustration in treating cancer patients over the years, many attempts have been made to develop diagnostic tests to guide treatment and monitor the effects of such treatment on metastasis or relapse.

One of the first attempts to develop a useful test for diagnostic oncology was the formulation of an immunoassay for carcinoembryonic antigen (CEA). This antigen appears on fetal cells and reappears on tumor cells in certain cancers. Extensive efforts have been made to evaluate the usefulness of testing for CEA as well as many other "tumor" antigens, such as prostate specific antigen (PSA), CA 15.3, CA 125, prostate-specific membrane antigen (PSMA), CA 27.29, p27 found in either tissue samples or blood as soluble cellular debris. These and other debris antigens are thought to be derived from destruction of both circulating and non-circulating tumor cells, and thus their presence may not always reflect metastatic potential, especially if the cells rupture while in an apoptotic state.

Additional tests used to predict tumor progression in cancer patients have focused upon correlating enzymatic indices like telomerase activity in biopsy-harvested tumor samples with an indication of an unfavorable or favorable prognosis (US 5,693,474; US 5,639,613). Assessing enzyme activity in this type of analysis can involve time-consuming laboratory procedures such as gel electrophoresis and Western blot analysis. Also, there are variations in the signal to noise and sensitivity in sample analysis based on the origin of the tumor. Despite these shortcomings, specific soluble tumor markers in blood can provide a rapid and efficient approach for developing a therapeutic strategy early in treatment. For example, detection of serum HER-2/neu and serum CA 15-3 in patients with metastatic breast cancer have been shown to be prognostic factors for metastatic breast cancer (Ali S.M., Leitzel K., Chinchilli V.M., Engle L., Demers L., Harvey H.A., Carney W., Allard J.W. and Lipton A., Relationship of Serum HER-2/neu and Serum CA 15-3 in Patients with Metastatic Breast Cancer, Clinical Chemistry, 48(8):1314-1320 (2002)). Increased HER-2/neu results in decreased response to hormone therapy, and is a significant prognostic factor in predicting responses to hormone receptor-positive metastatic breast cancer. Thus in malignancies where the HER-2/neu oncogene product is associated, methods have been described to monitor therapy or assess risks based on elevated levels (US 5,876,712). However in both cases, the base levels during remission, or even in healthy normals, are relatively high and may overlap with concentrations found in patients, thus requiring multiple testing and monitoring to establish patient-dependent baseline and cut-off levels.

In prostate cancer, PSA levels in serum have proven to be useful in early detection. When used with a follow-up physical examination (digital rectal exam) and biopsy, the PSA test has improved detection of prostate cancer at an early stage when it is best treated.

However, PSA or the related PSMA testing leaves much to be desired. For example, elevated levels of PSA weakly correlate with disease stage and appear not to be a reliable indicator of the metastatic potential of the tumor. This may be due in part to the fact that PSA is a component of normal prostate tissue and benign prostatic hyperplasia (BHP) tissue. Moreover, approximately 30% of patients with alleged localized prostate cancer and corresponding low serum PSA concentrations, may have metastatic disease (Moreno et al., Cancer Research, 52:6110 (1992)).

### Genetic markers:

One approach for determining the presence of malignant prostate tumor cells has been to test for the expression of messenger RNA from PSA in blood. This is being done through the laborious procedure of isolating all of the mRNA from the blood sample and performing reverse transcriptase PCR. No significant correlation has been described between the presence of shed tumor cells in blood and the ability to identify which patients would benefit from more vigorous treatment (Gomella LG., J of Urology, 158:326-337 (1997)). Additionally, false positives are often observed using this technique. There is an added drawback, which is that there is a finite and practical limit to the sensitivity of this technique based on the sample size. Typically, the test is performed on 10⁵ to 10⁶ cells separated from interfering red blood cells, corresponding to a practical lower limit of sensitivity of one tumor cell/0.1 ml of blood (about 10 tumor cells in one ml of blood) before a signal is detected. Higher sensitivity has been suggested by detecting hK2 RNA in tumor cells isolated from blood (US 6,479,263; US 6,235,486).

Qualitative RT-PCR based studies with blood-based nucleotide markers has been used to indicate that the potential for disease-free survival for patients with positive CEA mRNA in pre-operative blood is worse than that of patients negative for CEA mRNA (Hardingham J.E., Hewett P.J., Sage R.E., Finch J.L., Nuttal J.D., Kotasel D. and Dovrovic A., Molecular detection of blood-borne epithelial cells in colorectal cancer patients and in patients with benign bowel disease, Int. J. Cancer 89:8-13 (2000): Taniguchi T., Makino M., Suzuki K., Kaibara N., Prognostic significance of reverse transcriptase-polymerase chain reaction measurement of carcinoembryonic antigen mRNA levels in tumor drainage blood and peripheral blood of patients with colorectal carcinoma, Cancer 89:970-976 (2000)). The prognostic value of this endpoint is dependent upon CEA mRNA levels, which are also induced in healthy individuals by G-CSF, cytokines, steroids, or environmental factors. Hence, the CEA mRNA marker lacks specificity and is clearly not unique to circulating colorectal cancer cells. Other reports have implicated tyrosinase mRNA in peripheral blood and bone marrow as a marker for malignant melanoma in stage II-IV patients (Ghossein R.A., Coit D., Brennan M., Zhang Z.F., Wang Y., Bhattacharya S., Houghton A., and Rosai J., Prognostic significance of peripheral blood and bone marrow tyrosinase messenger RNA in malignant melanoma, Clin Cancer Res., 4(2):419-428 (1998)). Again using tyrosinase mRNA as a soluble tumor marker is subject to the previously cited limitations of soluble tumor antigens as indicators of metastatic potential and patient survival.

The aforementioned and other studies, while seemingly prognostic under the experimental conditions, do not provide for consistent data with a long follow-up period or at a satisfactory specificity. Accordingly, these efforts have proven to be somewhat futile as the appearance of mRNA for antigens in blood have not been generally predictive for most cancers and are often detected when there is little hope for the patient.

In spite of this, real-time reverse transcriptase-polymerase chain reaction (RT-PCR) has been the only procedure reported to correlate the quantitative detection of circulating tumor cells with patient prognosis. Real-time RT-PCR has been used for quantifying CEA mRNA in peripheral blood of colorectal cancer patients (Ito S., Nakanishi H., Hirai T., Kato T., Kodera Y., Feng Z., Kasai Y., Ito K., Akiyama S., Nakao A., and Tatematsu M., Quantitative detection of CEA expressing free tumor cells in the peripheral blood of colorectal cancer patients during surgery with real-time RT-PCR on a Light Cycler, Cancer Letters, 183:195-203 (2002)). Using Kaplan-Meier type analysis, disease free survival of patients with positive CEA mRNA in postoperative blood was significantly shorter than in cases that were negative for CEA mRNA. These results suggest that tumor cells were shed into the bloodstream (possibly during surgical procedures or from micro metastases already existing at the time of the operation), and resulted in poor patient outcomes in patients with colorectal cancer. The sensitivity of this assay provided a reproducibly detectable range similar in sensitivity to conventional RT-PCR. As mentioned, these detection ranges are based on unreliable conversions of amplified product to the number of tumor cells. The extrapolated cell count may include damaged CTC incapable of metastatic proliferation. Further, PCR-based assays are limited by possible sample contamination, along with an inability to quantify tumor cells. Most importantly, methods based on PCR, flowcytometry, cytoplasmic enzymes and circulating tumor antigens cannot provide essential morphological information confirming the structural integrity underlying metastatic potential of the presumed CTC and thus constitute functionally less reliable surrogate assays than the highly sensitive imaging methods embodied, in part, in this invention.

### Assessment of intact tumor cells in cancer detection and prognosis:

Detection of intact tumor cells in blood provides a direct link to recurrent metastatic disease in cancer patients who have undergone resection of their primary tumor. Unfortunately, the same spreading of malignant cells continues to be missed by conventional tumor staging procedures. Recent studies have shown that the presence of a single carcinoma cell in the bone marrow of cancer patients is an independent prognostic factor for metastatic relapse (Diel IJ, Kaufman M, Goerner R, Costa SD, Kaul S, Bastert G. Detection of tumor cells in bone marrow of patients with primary breast cancer: a prognostic factor for distant metastasis. J Clin Oncol, 10:1534-1539, 1992). But these invasive techniques are deemed undesirable or unacceptable for routine or multiple clinical assays compared to detection of disseminated epithelial tumor cells in blood.

An alternative approach incorporates immunomagnetic separation technology and provides greater sensitivity and specificity in the unequivocal detection of intact circulating cancer cells. This simple and sensitive diagnostic tool, as described (US6,365,362; US6,551,843; US6,623,982; US6,620,627; US6,645,731; WO 02/077604; WO03/065042; and WO 03/019141) is used in the present invention to correlate the statistical survivability of an individual patient based on a threshold level of greater than or equal to 5 tumor cells in 7.5 to 30 ml blood (1 to 2 tumor cells correspond to about 3000 to 4000 total tumor cells in circulation for a given individual).

Using this diagnostic tool, a blood sample from a cancer patient (WO 03/018757) is incubated with magnetic beads, coated with antibodies directed against an epithelial cell surface antigen as for example EpCAM. After labeling with anti-EpCAM-coated magnetic nanoparticles, the magnetically labeled cells are then isolated using a magnetic separator. The immunomagnetically enriched fraction is further processed for downstream immunocytochemical analysis or image cytometry, for example, in the Cell Spotter^{®} System (Immunicon Corp., PA). The magnetic fraction can also be used for downstream immunocytochemical analysis, RT-PCR, PCR, FISH, flowcytometry, or other types of image cytometry.

One embodiment of the present invention includes image cytometry after immunomagnetic selection and separation to highly enrich and concentrate any epithelial cells present in whole blood samples. The captured cells are detectably labeled with a leukocyte specific marker and with one or more tumor cell specific fluorescent monoclonal antibodies to allow identification and enumeration of the captured CTC's as well as unequivocal instrumental or visual differentiation from contaminating non-target cells. At an extraordinary sensitivity of 1 or 2 epithelial cells per 7.5- 30 ml of blood, this assay allows tumor cell detection even in the early stages of low tumor mass. The embodiment of the present invention is not limited to image cytometry, but includes any isolation and imaging protocol of comparable sensitivity and specificity.

Currently available prognostic protocols have not demonstrated a consistently reliable means for correlating CTCs to predict progression free- or overall survival in patients with cancers such as metastatic breast cancer (MBC). Thus, there is a clear need for accurate detection of cancer cells with metastatic potential, not only in MBC but in metastatic cancers in general. Moreover, this need is accentuated by the need to select the most effective therapy for a given patient. We previously described a method for predicting progression free or overall survival in patients with the first follow-up after diagnosis (WO 2004/076643). In this method, >5 circulating tumor cells (CTCs) in 7.5ml of blood is associated with poor clinical outcome. In the present invention, we provide a method for predicting progression free and overall survival at each follow-up time point during therapy of metastatic breast cancer patients.

Massimo Cristofanilli et al: "Circulating tumor cells: a novel prognosis factor for newly diagnosed metastatic breast cancer", Journal of Clinical Oncology: Official Journal of the American Society of Clinical Oncology, vol 23, no. 7,1 March 2005, pages 1420-1430, describes a statistical analysis of circulating tumour cells as a prognostic factor for newly diagnosed metastatic breast cancer.

Massimo Cristofanilli et al: "Circulating tumor cells, disease progression, and survival in metastatic breast cancer", New England Journal of Medicine, vol 351, no. 8,19 August 2004, pages 781-791, describes predicting survival in metastatic breast cancer patients by a statistical analysis of the level of circulating tumour cells.

US 2002/098535 A1 describes isolating immunomagnetically separated cancer cells and characterising them to determine whether cancer is progressing, stable or being terminated.

WO 99/41613 A1 states that when rare cells in a mixed cell-population are detected and enumerated, the severity of the disease state is greater the greater the number of rare cells present.

US 5993388 A describes determining survival rates in prostate cancer patients using a Kaplan-Meier analysis of PSA.

### Summary of the Invention

The present invention is a method and an analysis system for overall survival prognosis, according to claim 1, 8, 9 or 16 incorporating diagnostic tools, such as immunomagnetic enrichment and image cytometry, in assessing time to disease progression, survival, and response to therapy based upon the absolute number, change, or combinations of both of circulating epithelial cells in patients with metastatic cancer at any time during therapy to provide an accurate indication of emerging rapid disease progression and mortality for metastatic breast cancer patients. The system immunomagnetically concentrates epithelial cells, fluorescently labels the cells, identifies and quantifies CTCs for positive enumeration. The statistical analysis of the cell count predicts survival.

More specifically, the present invention provides the analysis system and method for assessing patient survival, the time to disease progression, and response to therapy in MBC. Prediction of survival is based upon a threshold comparison of the number of circulating tumor cells in blood with time to death and disease progression. Statistical analysis of long term follow-up studies of patients diagnosed with cancer established a threshold for the number of CTC found in blood and prediction of survival. An absence of CTCs is defined as fewer than 5 morphologically intact CTCs. The presence or absence of CTCs to predict survival is useful in making treatment choices.

For example, the absence of CTC's in a woman previously untreated for metastatic breast cancer could be used to select hormonal therapy vs chemotherapy with less side effects and higher quality of life. In contrast, the presence of CTC's could be used to select chemotherapy which has higher side effects but may prolong survival more effectively in a high risk population. Thus, the invention has a prognostic role in the detection of CTC's in women with metastatic breast cancer.

### Brief Description of the Drawings

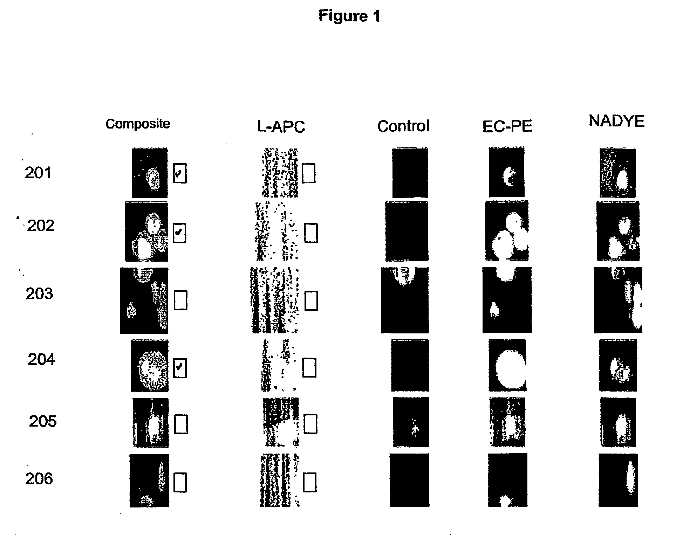

**Figure 1****:** Cell Spotter® fluorescent analysis profile used to confirm objects captured as tumor cells. Check marks signify a positive tumor cell based on the composite image. Composite images are derived from the positive selection for Epithelial Cell Marker (EC-PE) and for the nuclear dye (NADYE). A negative selection is also needed for the leukocyte marker (L-APC) and for control (CNTL).

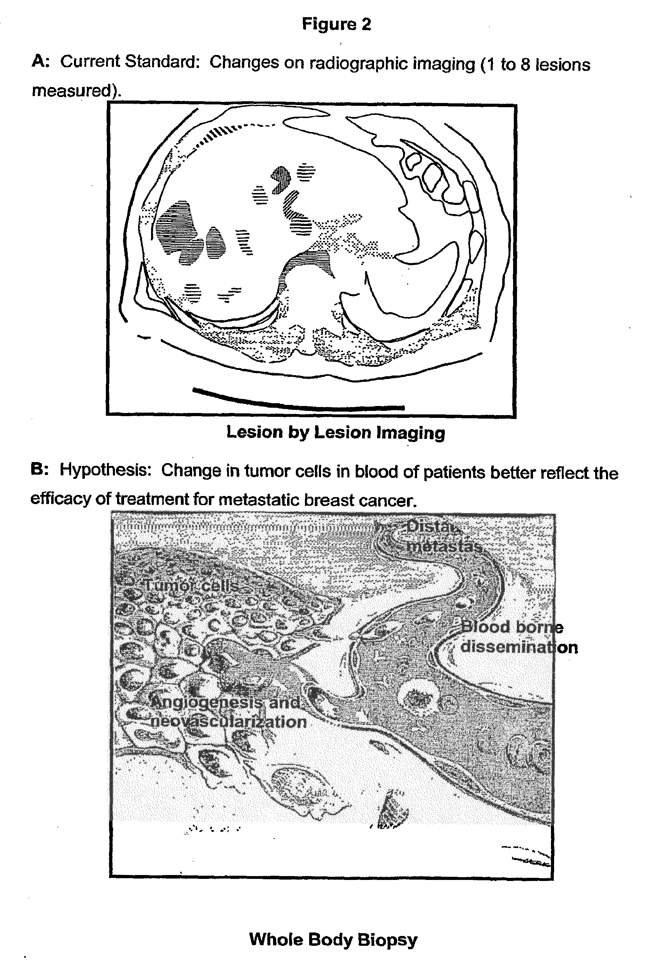

**Figure 2****:** Comparison of diagnostic methods to measure changes in tumor status. Shown is the current standard of physical measurement of discrete lesions using radiographic imaging (Panel A). A model illustrating the ability to assess changes in metastatic cancer burden by counting the numbers of CTC in blood is shown (Panel B).

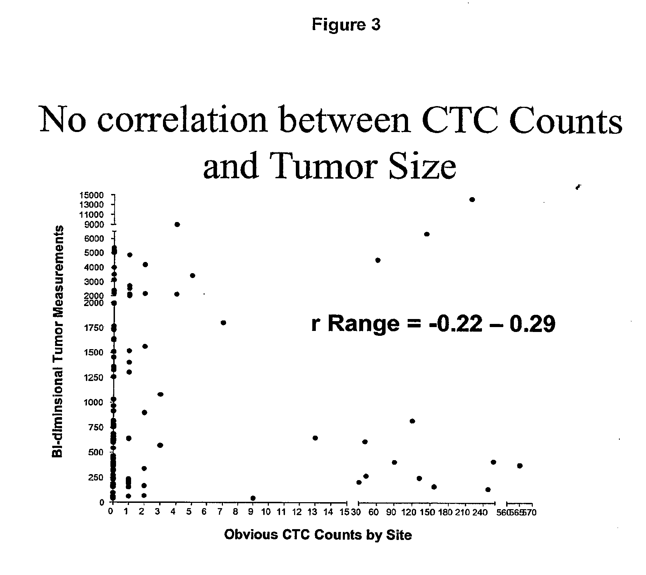

**Figure 3****:** Lack of correlation between the number of CTC's and tumor size in 69 patients.

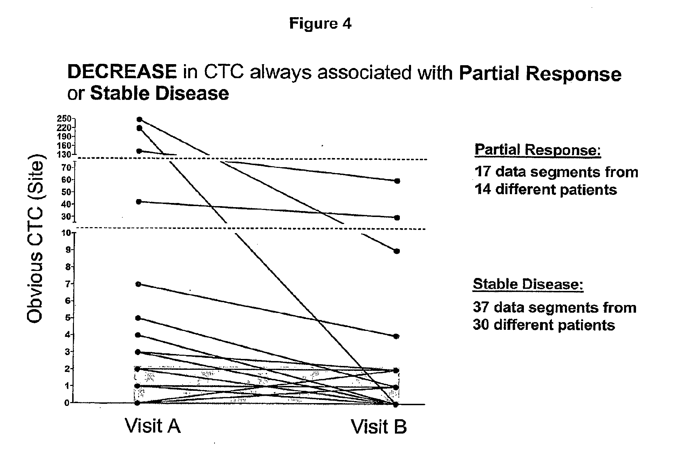

**Figure 4****:** Changes in the numbers of CTC's in patients with a partial response to therapy or with a stable disease state. CTC's either decreased or remained undetectable in all cases.

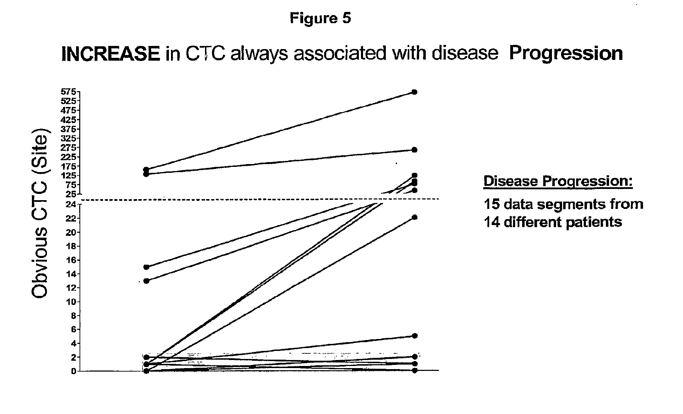

**Figure 5****:** Changes in the numbers of CTC's in patients with disease progression. CTCs either increased or remained undetectable with disease progression.

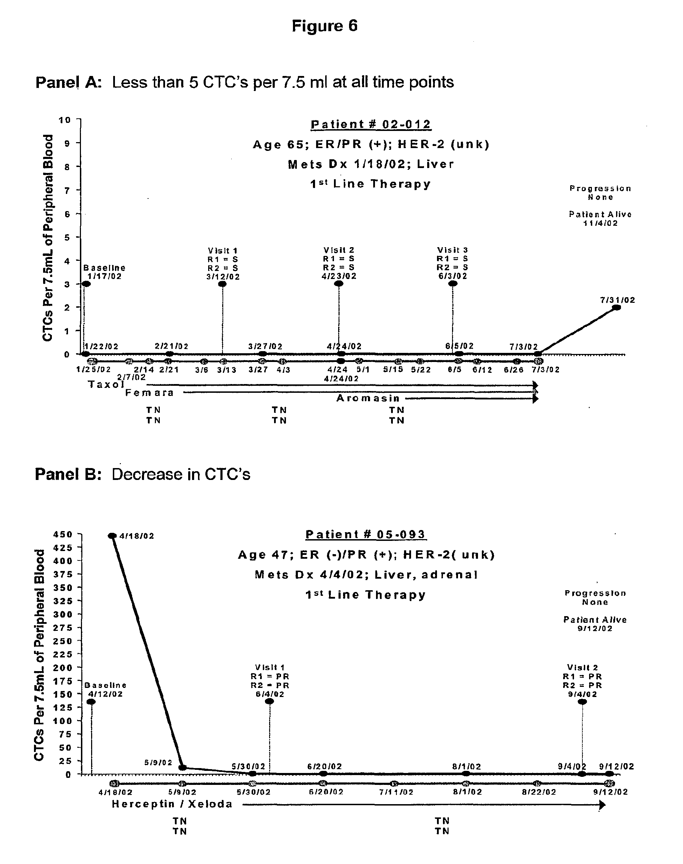

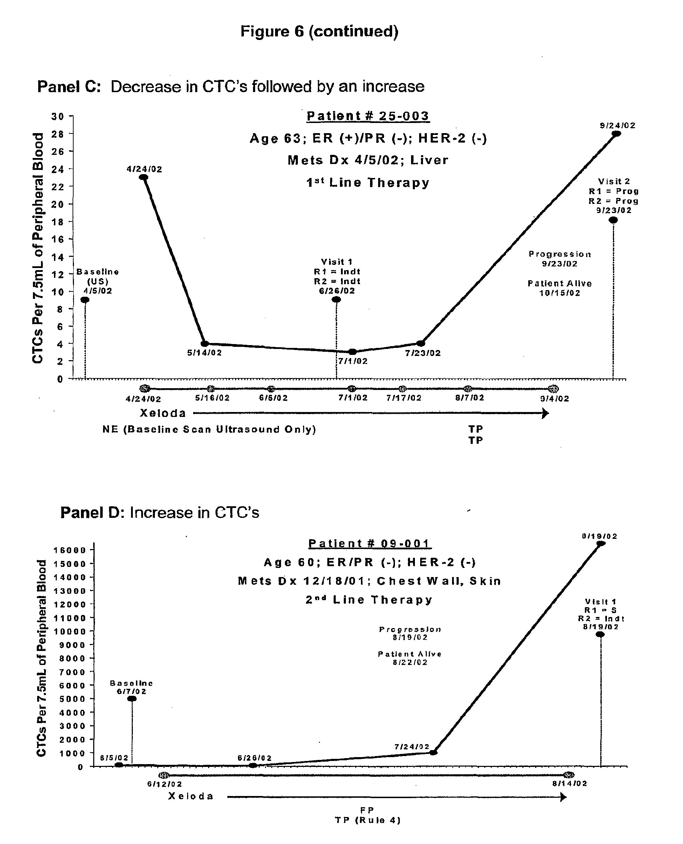

**Figure 6****:** Patient trends in the number of CTC's. Panel A shows a typical patient with less than 5 CTC's per 7.5 ml of blood. Panel B shows a typical patient with a decrease in CTC's during the course of therapy. Panel C shows a typical patient with a decrease in CTC's followed by an increase. Panel D shows a typical patient with an increase in CTC's.

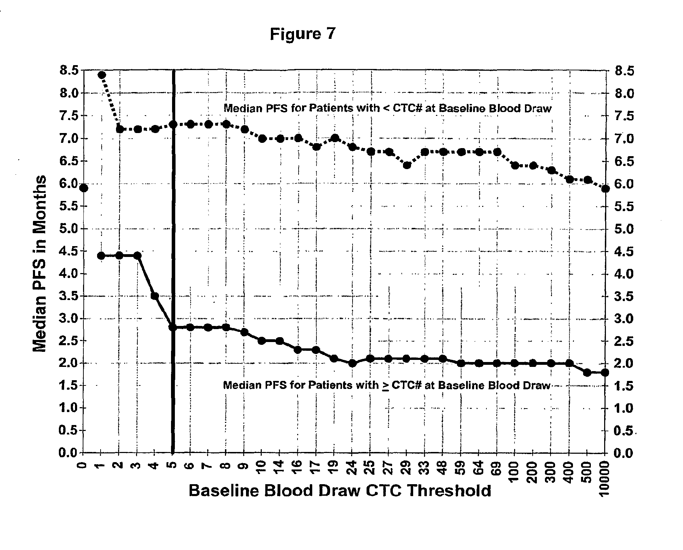

**Figure 7****:** Determination of an optimal CTC cutoff for distinguishing MBC patients with rapid progression. Analysis was performed using the CTC numbers obtained at baseline from the 102 patients included in the training set. Median PFS of patients with greater than or equal to the selected number of CTC in 7.5mL of blood is indicated by the solid line and median PFS of patients with less than the selected CTC level is indicated by the dashed line. Median PFS decreased as CTC increased and reached a plateau that leveled off at 5 CTC (indicated by the vertical line). The black dot indicates the median PFS of∼5.9 months for all 102 patients. The selected cutoff of ≥5 CTC/7.5mL was used in all subsequent analyses.

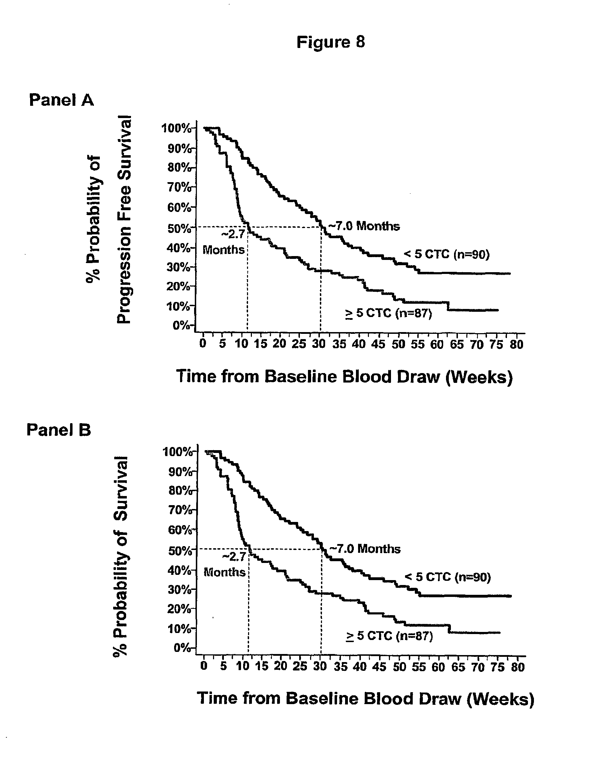

**Figure 8****:** The predictive value of baseline CTC for PFS and OS. Probabilities of PFS and OS of MBC patients with <5 (black line) and ≥5 (gray line) CTC's in 7.5mL of blood using the baseline blood draw prior to initiation of a new line of therapy are shown. PFS and OS were calculated from the time of the baseline blood draw.. Panel A: the probability of PFS using the baseline CTC count (n=177, log-rank p=0.0001, CoxHR =1.95, chi² =15.33, p= 0.0001). Panel B: the probability of OS using the baseline CTC count (n=102, log-rank p=0.0003, CoxHR =3.98, chi² =12.64, p= 0.0004).

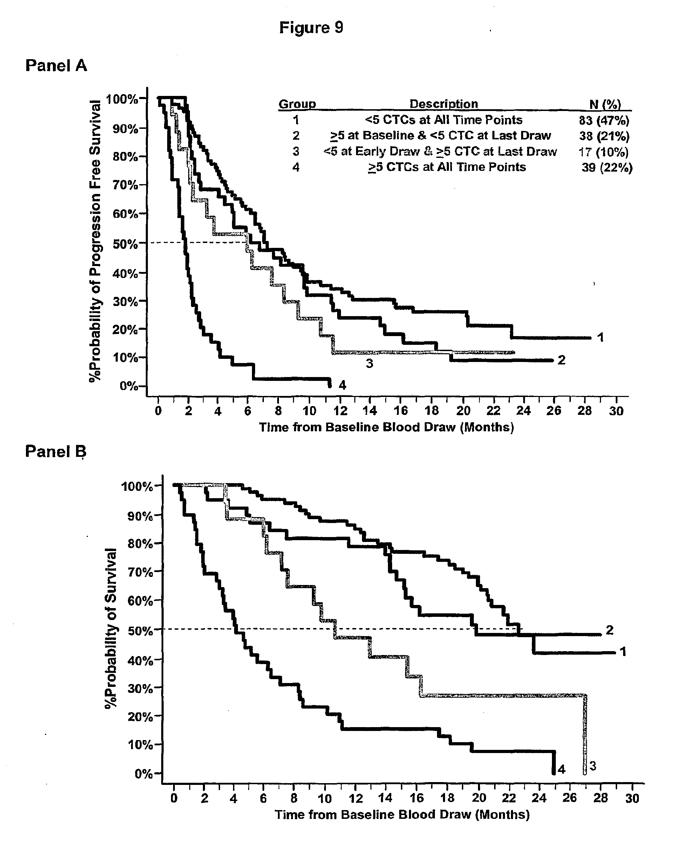

**Figure 9****:** Kaplan-Meier plot of CTC levels prior to and up to 15-20 weeks after the initiation of therapy to predict time to clinical progression or death (Figure 9A) or the time to death (Figure 9B) from the date of the baseline blood draw in 177 patients with metastatic breast cancer. Four different groups of patients are compared: Group 1, 83 (47%) patients with <5 CTCs at all blood draw time points; Group 2, 38 (21 %) patients with ≥5 CTCs prior to the initiation of therapy but who had decreased to <5 CTCs at the time of their final blood draw; Group 3, 17 (10%) patients with <5 CTCs prior to the initiation of therapy who increased to ≥5 CTCs at the time of their last blood draw; and Group 4, 39 (22%) patients with ≥5 CTCs at all blood draw time points

### Detailed Description of the Invention

The object of this invention provides for the detection of circulating tumor cells as an early prognostic indicator of patient survival.

Under the broadest aspect of the invention, there is no limitation on the collection and handling of samples as long as consistency is maintained. Accordingly, the cells can be obtained by methods known in the art.

While any effective mechanism for isolating, enriching, and analyzing CTCs in blood is appropriate, one method for collecting circulating tumor cells combines immunomagnetic enrichment technology, immunofluorescent labeling technology with an appropriate analytical platform after initial blood draw. The associated test has the sensitivity and specificity to detect these rare cells in a sample of whole blood and to investigate their role in the clinical course of the disease in malignant tumors of epithelial origin. From a sample of whole blood, rare cells are detected with a sensitivity and specificity to allow them to be collected and used in the diagnostic assays of the invention, namely predicting the clinical course of disease in malignant tumors.

With this technology, circulating tumor cells (CTC) have been shown to exist in the blood in detectable amounts. This created a tool to investigate the significance of cells of epithelial origin in the peripheral circulation of cancer patients (Racila E., Euhus D., Weiss A.J., Rao C., McConnell J., Terstappen L.W.M.M. and Uhr J.W., Detection and characterization of carcinoma cells in the blood, Proc. Natl. Acad. Sci. USA, 95:4589-4594 (1998)). This study demonstrated that these blood-borne cells might have a significant role in the pathophysiology of cancer. Having a detection sensitivity of 1 epithelial cell per 5 ml of blood, the assay incorporates immunomagnetic sample enrichment and fluorescent monoclonal antibody staining followed by flowcytometry for a rapid and sensitive analysis of a sample. The results show that the number of epithelial cells in peripheral blood of eight patients treated for metastatic carcinoma of the breast correlate with disease progression and response to therapy. In 13 of 14 patients with localized disease, 5 of 5 patients with lymph node involvement and 11 of 11 patients with distant metastasis, epithelial cells were found in peripheral blood. The number of epithelial cells was significantly larger in patients with extensive disease.

The assay was further configured to an image cytometric analysis such that the immunomagnetically enriched sample is analyzed by image cytometry (see Example 1). This is a fluorescence-based microscope image analysis system, which in contrast with flowcytometric analysis permits the visualization of events and the assessment of morphologic features to further identify objects.

Automated fluorescence microscopic system, used for automated enumeration of isolated cells from blood, allows for an integrated computer controlled fluorescence microscope and automated stage with a magnetic yoke assembly that will hold a disposable sample cartridge. The magnetic yoke is designed to enable ferrofluid-labeled candidate tumor cells within the sample chamber to be magnetically localized to the upper viewing surface of the sample cartridge for microscopic viewing. Software presents suspect cancer cells, labeled with antibodies to cytokeratin and having epithelial origin, to the operator for final selection.

While isolation of tumor cells can be accomplished by any means known in the art, one embodiment uses a cell stabilization and permeabilization procedure for isolating tumor cells in 7.5 ml of whole blood. Epithelial cell-specific magnetic particles are added and incubated for 20 minutes. After magnetic separation, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination. Cells are identified automatically and candidate circulating tumor cells presented to the operator for checklist enumeration. An enumeration checklist consists of predetermined morphologic criteria constituting a complete cell (see example 1).

The diagnostic potential of immunomagnetic enrichment and image cytometry, together with the use of intact circulating tumor cells as a prognostic factor in cancer survival, can provide a rapid and sensitive method for determining appropriate treatment. Accordingly in the present invention, the analysis system and method are provided for the rapid enumeration and characterization of tumor cells shed into the blood in metastatic and primary patients for prognostic assessment of survival potential.

The methods of the invention are useful in assessing a favorable or unfavorable survival, and even preventing unnecessary therapy that could result in harmful side-effects when the prognosis is favorable.

The following examples illustrate the predictive and prognostic value of CTC's in blood from patients. Note, the following examples are offered by way of illustration and are not in any way intended to limit the scope of the invention.

### • Example 1

### Enumeration of circulating cytokeratin positive cells using CellPrep™

Cytokeratin positive cells are isolated by a cell preservative system using a 7.5 ml sample of whole blood. Epithelial cell-specific immunomagnetic fluid is added and incubated for 20 minutes. After magnetic separation for 20 minutes, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample for 15 minutes. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination. Cells are identified automatically; control cells are enumerated by the system, whereas the candidate circulating tumor cells are presented to the operator for enumeration using a checklist as shown (Figure 1).

### • Example 2

### Assessment of the tumor load: Comparison between radiographic image analysis and the absolute number of CTC's.

Radiographic measurements of metastatic lesions are currently used to assess tumor load in cancer patients with metastatic disease. In general, the largest lesions are measured and summed to obtain a tumor load. An example of a bidimensional measurement of a liver metastasis in a breast cancer patient is illustrated in Figure 2A. A model depicting the necessity for measuring tumor load in the blood stream is illustrated in Figure 2B as a measurement of the actual active tumor load, and thus a better measure of the overall activity of the disease. To determine whether or not the absolute number of CTC's correlated with the dimension of the tumor measured by imaging a prospective study in patients with MBC was performed.

Image cytometry was used to enumerate CTC's in 7.5 ml of blood from 69 patients with measurable MBC. Tumor load was assessed by bidimensional radiographic measurements of up to 8 measurable lesions before initiation of therapy. The tumor load was determined by addition of the individual measurements (mm²). CTC's were enumerated in blood drawn before initiation of therapy.

Figure 3 shows the number of CTC's in 7.5 ml versus the bidimensional sums of tumor measurements in the 69 patients. From Figure 3, there is no correlation between the size of the tumor and the absolute number of tumor cells in the blood. Some patients with large tumors as measured by imaging have low numbers of CTC's and vice versa.

Thus, tumor burden as measured by radiographic imaging does not correlate with the absolute number of tumor cells present in the blood.

### Example 3

### Assessment of the tumor load: Comparison between changes in the radiographic image and changes in the absolute number of CTC's.

Radiographic imaging is the current standard to assess whether a particular disease is responding, stabilizing, or progressing to treatment. The interval between radiographic measurements must be at least 3 months in order to give meaningful results. Consequently, a test that could predict response to therapy earlier during the treatment cycle would improve the management of patients treated for metastatic diseases, potentially increase quality of life and possibly improve survival. In this study, patients starting a new line of treatment for MBC were assessed to determine whether a change in the number of CTC's correlated with a change in patient status as measured by imaging.

This imaging system was used to enumerate CTC's in 7.5 ml of blood in MBC patients about to start a new therapy, and at various time points during the treatment cycle. Radiographic measurements were made before initiation of therapy, 10-12 weeks after initiation of therapy and after completion of the treatment cycle (approximately 6 months after initiation of therapy), or at the time the patient progressed on therapy, whichever came first.

From image analysis, a partial response was found in 14 patients (17 data segments). CTC's either decreased or remained undetectable in all cases (see Figure 4). Stable disease by imaging was found in 30 patients (37 data segments). CTC's either decreased or remained not detectable in all cases (see Figure 4). Disease progression by imaging was found in 14 patients (15 data segments). CTC's increased in 7 of 15 cases. No CTC's were detected at either time point in the other 8 cases.

An increase in CTC's was only observed in patients with disease progression (100%). A decrease in CTC's was only observed in patients with a partial response or stable disease (100%). In patients with a partial response or stable disease, no CTC's were detected at both time points in 54 of 61 cases (89%).

### • Example 4

### Trends in the number of CTC's in patients treated for MBC as a guide to treatment.

A study in patients with MBC was performed to determine whether or not clear trends in changes of the number of CTC could be observed in patients treated for MBC, and whether or not simple rules could be applied to such trends in order to guide the treating physician in optimization of the treatment of patients with MBC.

Image cytometry was used to enumerate CTC's in 7.5 ml of blood. 81 patients, starting a new line of therapy for MBC, were enrolled in the study. CTC's were enumerated in blood drawn before initiation of therapy and at approximately every month thereafter.

Clear trends in the number of CTC's were observed in 76 of 81 (94%) patients. During the course of therapy, the number of CTC's was not detectable or remained below 5 CTC per 7.5 ml of blood in 50% of the patients. A typical example is shown in Figure 6A. The number of CTC's decreased during the course of therapy in 22% of the patients. A typical example is shown in Figure 6B. A decrease in the number of CTC's followed by an increase during the course of therapy was observed in 6% of the patients. A typical example is shown in Figure 6C. The number of CTC's increased during the course of therapy in 16% of the patients. A typical example is shown in Figure 6D. In 42 instances, 2 blood samples were prepared and analyzed at the time of each blood draw. Results using the first tubes drawn at the initial timepoint and the first tube drawn at the follow-up time point point were compared to results using the second tubes drawn at each timepoint. In only one of those cases, the change in the number of CTC's was different between the first tubes drawn and the second (or duplicate) tubes drawn (98% agreement). In this case, both tubes from the first blood draw had 0 CTC's, whereas for the second blood draw, one tube had 5 CTC(below the cut off) and the second tube had 6 CTC (above the cut off). In comparison to the reproducibility of CTC measurements, inter-reader variability of radiographic imaging when the same films were read by two different expert radiologists resulted in an agreement of only 81%. More over, the agreement between the two radiologists in a set of 146 imaging segments was 85% when Progression versus non progression was measured and decreased to only 58% when Progression, Stable Disease and Partial response were measured. In contrast, analysis of CTC measurement was performed on the same data set by two different technologists, resulting in 100% agreement.

Thus, detection and monitoring CTC in patients treated for MBC is a more reproducible procedure to measure response to therapy than radiographic imaging.

### • Example 5

### Prediction of PFS and OS before initiation of therapy.

A study to correlate CTC levels before initiation of therapy with progression-free survival (PFS) and overall survival (OS) was performed whereby a threshold value of ≥5 CTC's/7.5 ml was used.

177 patients with measurable MBC were tested for CTC's in 7.5 ml of blood before starting a new line of treatment and at subsequent monthly intervals for a period of up to six months. Patients entering into any type of therapy and any line of therapy were included in the trial. Disease progression or response was assessed by the physicians at the sites for each patient.

As shown in Figure 7, median PFS decreased as CTC levels increased and reached a plateau that leveled off at 5 CTC's (vertical line). The median PFS was approximately 5.9 months for all patients (black dot). Based on the change in median PFS for positive patients and the Cox Hazard's ratio, a cutoff of 2≥ 5 CTC's was used for all subsequent analysis.

Figure 8 shows a Kaplan Meier analysis of Progression Free Survival (PFS) and Overall Survival (OS) using the number of CTC measured in the baseline blood draws. In the 177 patients, the median PFS time was approximately 5.0 months. The patients with ≥ 5 CTC's/7.5 ml of blood at baseline had a significantly shorter PFS than patients with < 5 CTC's (approximately 2.7 months vs. 7.0 months, respectively). Overall survival (OS) reflected the same trend with a median OS of 10.1 months vs. > 18 months for patients with ≥ 5 CTC's vs. < 5 CTC's, respectively.

The measurement of the number of CTC prior to initation of a new line of therapy predicts the time until patients progress on their therapy, and predicts survival time. Because of this predictive ability, detection and measurement of CTC's at baseline provides information to physicians that will be useful in the selection of appropriate treatment. In addition, the ability to stratify patients into high and low risk groups in terms of PFS and OS may be very useful to select appropriate patients for entry into therapeutic trials. For novel drugs with potentially high toxicity, patients with poor prognostic factors may be the more appropriate target population. In contrast, drugs with minimal toxicity and promising therapeutic efficacy may be more appropriately targeted toward patients with favorable prognostic factors.

### • Example 6

### Prediction of PFS and OS at each follow-up time point during therapy of metastatic breast cancer patients.

CTCs were assessed serially over the course of treatment at additional specified intervals. The results demonstrated that assessment of CTC levels at "all" subsequent follow-up time points, accurately and reproducibly predicted the clinical outcome. Patients who converted from elevated CTCs to non-elevated levels (<5CTC/7.5mL) exhibited PFS and OS similar to those whose CTCs were never elevated. This would imply that patients with <5 CTCs appear to either be responding to treatment and/or have relatively indolent disease. In contrast, OS of patients who converted from non-elevated CTC levels to elevated CTC levels decreased, but was not as short as the OS of patients that always exhibited elevated CTC levels.

CTCs were enumerated in 177 MBC patients prior to the initiation of a new course of therapy and 3-5, 6-8, 9-14, and 15-20 weeks after the initiation of therapy. Progression free survival (PFS) and overall survival (OS) times were calculated from the dates of each follow-up blood draw. Kaplan-Meier plots and survival analyses were performed using a threshold of ≥5 CTCs/7.5mL at each blood draw.

Median PFS times for patients with <5 CTC at the five blood draw time points were 7.0, 6.1, 5.6, 7.0, and 6.0 months, respectively. For patients with ≥5 CTC, median PFS was significantly shorter at these time points: 2.7, 1.3, 1.4, 3.0, and 3.6 months, respectively. Median OS for patients with <5 CTC at baseline and the five blood draw time points were all >18.5 months. For patients with ≥5 CTC, median OS was significantly shorter at these time points: 10.9, 6.3, 6.3, 6.6, and 6.7 months, respectively. Median PFS (Figure 9A) and OS (Figure 9B) times at baseline and up to 9-14 weeks after the initiation of therapy were statistically significantly different.

Detection of elevated CTCs at any time during therapy is an accurate indication of emerging rapid disease progression and mortality for MBC patients Accordingly, CTC's must decline to below 5 at any time point in the clinical course of a patient with metastatic breast cancer to maximize PFS and OS, and to maximize the benefit associated with therapy.

## Claims

1. A method for overall survival prognosis in patients at any point during therapy of metastatic breast cancer patients comprising:
a) enriching a fraction of a blood specimen having a mixed cell population suspected of containing circulating tumour cells (CTCs), said fraction containing said CTCs;
b) confirming structural integrity of said CTCs to be intact; and
c) analyzing said intact CTCs at any time during follow-up treatment of the patient to determine patient survival, wherein said analyzing correlates intact CTC enumeration of said patient with said overall survival prognosis based upon a predetermined statistical association, said analyzing being based upon a measurement of CTC number relative to a threshold number of 5 circulating tumour cells per 7.5 ml of blood, said measurement above or equal to said threshold being indicative of a lower said survival prognosis.

2. A method as claimed in claim 1, wherein said circulating CTCs are epithelial cells.

3. A method as claimed in claim 1, wherein said fraction is obtained by immunomagnetic enrichment, wherein said specimen is mixed with magnetic particles coupled to a biospecific ligand which specifically binds to said CTCs, to the substantial exclusion of other populations and subjecting specimen-magnetic particle mixture to a magnetic field to produce a cell suspension enriched in magnetic particle-bound CTCs.

4. A method as claimed in claim 1, wherein said biospecific ligand is an antibody directed against an epithelial cell surface antigen.

5. A method as claimed in claim 4, wherein said CTCs have EpCAM as said epithelial cell surface antigen.

6. A method as claimed in claim 1, wherein said structural integrity is determined by a procedure selected from a group consisting of immunocytochemical procedures, RT-PCR procedures, PCR procedures, FISH procedures, flowcytometry procedures, image cytometry procedures, and combinations thereof.

7. A method as claimed in claim 1, wherein said analyzing is based upon a change in said intact CTC enumeration occuring during the last follow-up period to indicate said survival prognosis.

8. A CTC analysis system for assessing overall survival in metastatic breast cancer patients at any point during therapy, when used in the method of claim 1, comprising:
a) means for stabilizing cells in a blood specimen from said patient, said means preserving characteristic determinants of CTCs in said specimen, wherein said stabilizing means is selected from the group consisting of anti-coagulating agents, stabilizing agents, and combinations thereof;
b) means for enriching a fraction of said specimen, said fraction containing intact CTCs, wherein said enriching means is selected from the group consisting of immunomagnetic, density centrifugation, and combinations thereof;
c) means for confirming structural integrity of said intact CTCs, wherein said confirming means is selected from the group consisting of immunocytochemical means, RT-PCR means, PCR means, FISH means, flowcytometry means, image cytometry means, and combinations thereof; and
d) means for analyzing said intact CTCs at any time during follow-up treatment of the patient, wherein said means correlates said intact CTC enumeration with said overall survival based upon a predetermined statistical association, said analyzing being based upon a measurement of CTC number relative to a threshold number of 5 circulating tumour cells per 7.5 ml of blood, said measurement above or equal to said threshold being indicative of a lower said survival prognosis, wherein said analysis means is selected from the group consisting of Kaplan-Meier analysis means, Cox proportional hazards regression analysis means, and combinations thereof.

9. A method for assessing time to disease progression in metastatic breast cancer patients at any time point during therapy comprising:
a) enriching a fraction of a blood specimen having a mixed cell population suspected of containing CTCs,, said fraction containing said CTCs;
b) confirming structural integrity of said CTCs to be intact; and
c) analyzing said intact CTCs at any time during follow-up treatment of the patient to determine time to disease progression, wherein said analyzing correlates intact CTC enumeration of said patient with said time to disease progression based upon a predetermined statistical association, said analyzing being based upon a measurement of CTC number relative to a threshold number of 5 circulating tumour cells per 7.5 ml of blood, said measurement above or equal to said threshold being indicative of a lower said time to disease progression.

10. A method as claimed in claim 9, wherein said circulating CTCs are epithelial cells.

11. A method as claimed in claim 9, wherein said fraction is obtained by immunomagnetic enrichment, wherein said specimen is mixed with magnetic particles coupled to a biospecific ligand which specifically binds to said CTCs, to the substantial exclusion of other populations and subjecting specimen-magnetic particle mixture to a magnetic field to produce a cell suspension enriched in magnetic particle-bound CTCs.

12. A method as claimed in claim 9, wherein said biospecific ligand is an antibody directed against an epithelial cell surface antigen.

13. A method as claimed in claim 9, wherein said CTCs have EpCAM as said epithelial cell surface antigen.

14. A method as claimed in claim 9, wherein said structural integrity is determined by a procedure selected from a group consisting of immunocytochemical procedures, RT-PCR procedures, PCR procedures, FISH procedures, flowcytometry procedures, image cytometry procedures, and combinations thereof.

15. A method as claimed in claim 9, wherein said analyzing is based upon a change in said intact CTC enumeration during the last follow-up period to indicate said time to disease progression.

16. A CTC analysis system for assessing time to disease progression in a metastatic breast cancer patient at any time during therapy, when used in the method of claim 9, said CTC analysis system comprising:
a) means for stabilizing cells in a blood specimen from said patient, said means preserving characteristic determinants of CTCs in said specimen, wherein said stabilizing means is selected from the group consisting of anti-coagulating agents, stabilizing agents, and combination thereof;
b) means for enriching a fraction of said specimen, said fraction containing intact CTCs, wherein said enriching means is selected from a group consisting of immunomagnetic, density centrifugation, and combinations thereof;
c) means for confirming structural integrity of said intact CTCs, wherein said confirming means is selected from the group consisting of immunocytochemical means, RT-PCR means, PCR means, FISH means, flowcytometry means, image cytometry means, and combinations thereof; and
d) means for analyzing said intact CTCs at any time during follow-up treatment of the patient, wherein said means correlates said intact CTC enumeration with said time to disease progression based upon a predetermined statistical association, said analyzing being based upon a measurement of CTC number relative to a threshold number of 5 circulating tumour cells per 7.5 ml of blood, said measurement above or equal to said threshold being indicative of a lower said time to disease progression, wherein said analysis means is selected from the group consisting of Kaplan-Meier analysis means, Cox proportional hazards regression analysis means, and combinations thereof.

## Patentansprüche

1. Verfahren zur Prognose des Gesamtüberlebens bei Patienten zu irgendeinem Zeitpunkt während der Behandlung von Patienten mit metastasierendem Brustkrebs, umfassend:
a) Anreichern einer Fraktion einer Blutprobe mit einer gemischten Zellpopulation, die unter Verdacht steht zirkulierende Tumorzellen (circluating tumor cells, CTCs) zu enthalten, wobei die Fraktion die CTCs enthält;
b) Bestätigen, dass die strukturelle Integrität der CTCs intakt ist; und
c) Analysieren der intakten CTCs zu irgendeinem Zeitpunkt während der Nachbehandlung des Patienten, um das Überleben des Patienten zu bestimmen, wobei das Analysieren die Auszählung intakter CTCs des Patienten mit der Prognose des Gesamtüberlebens, die auf einem vorbestimmten statistischen Zusammenhang basiert, korreliert, wobei das Analysieren auf einer Messung der Anzahl von CTCs bezogen auf einen Schwellenwert von 5 zirkulierenden Tumorzellen pro 7,5 mL Blut basiert, wobei der Messwert oberhalb oder gleich diesem Schwellenwert auf eine niedrigere Überlebensprognose hinweist.

2. Verfahren nach Anspruch 1, wobei die zirkulierenden CTCs Ephithelzellen sind.

3. Verfahren nach Anspruch 1, wobei die Fraktion durch immunomagnetische Anreicherung erhalten wird, wobei die Probe mit magnetischen Teilchen, die an einen biospezifischen Liganden gebunden sind, der spezifisch an die CTCs bindet, gemischt wird, zum wesentlichen Ausschluss anderer Populationen und Aussetzen des Gemisches aus Probe und magnetischen Partikeln einem Magnetfeld, um eine Zellsuspension herzustellen, welche an CTCs angereichert ist, die an magnetische Partikel gebundenen sind.

4. Ein Verfahren nach Anspruch 1, wobei der biospezifische Ligand ein gegen ein Oberflächenantigen von Epithelzellen gerichteter Antikörper ist.

5. Verfahren nach Anspruch 4, wobei die CTCs als das Oberflächenantigen von Epithelzellen EpCAM aufweisen.

6. Verfahren nach Anspruch 1, wobei die strukturelle Integrität durch ein Verfahren bestimmt wird, das aus einer Gruppe bestehend aus immunzytochemischen Verfahren, RT-PCR-Verfahren, PCR-Verfahren, FISH-Verfahren, Durchflusszytometrie-Verfahren, Bildzytometrie-Verfahren und Kombinationen davon ausgewählt ist.

7. Verfahren nach Anspruch 1, wobei das Analysieren auf einer Änderung bei der Auszählung der intakten CTCs basiert, die während des letzten Nachbeobachtungszeitraums auftritt, um auf die Überlebensprognose hinzuweisen.

8. CTC-Analysesystem zur Beurteilung des Gesamtüberlebens bei Patienten mit metastasierendem Brustkrebs zu irgendeinem Zeitpunkt während der Behandlung, wenn in dem Verfahren nach Anspruch 1 verwendet, umfassend:
a) Mittel zum Stabilisieren von Zellen in einer Blutprobe des Patienten, wobei das Mittel charakteristische Determinanten der CTCs in der Probe bewahrt, wobei das Stabilisierungsmittel aus der Gruppe bestehend aus anti-koagulierenden Mitteln, Stabilisatoren und Kombinationen davon ausgewählt ist;
b) Mittel zum Anreichern einer Fraktion der Probe, wobei die Fraktion intakte CTCs enthält, wobei das Anreicherungsmittel aus der Gruppe bestehend aus immunomagnetischen Mitteln, Dichtezentrifugation und Kombinationen davon ausgewählt ist;
c) Mittel zur Bestätigung der strukturellen Integrität der intakten CTCs, wobei das Bestätigungsmittel aus der Gruppe bestehend aus immunzytochemischen Mitteln, RT-PCR-Mitteln, PCR-Mitteln, FISH-Mitteln, Durchflusszytometrie-Mitteln, Bildzytometrie-Mittel und Kombinationen davon ausgewählt ist; und
d) Mittel zum Analysieren der intakten CTCs zu irgendeinem Zeitpunkt während der Nachbehandlung des Patienten, wobei das Mittel die Auszählung intakter CTCs mit dem Gesamtüberleben, welches auf einem vorbestimmten statistischen Zusammenhang basiert, korreliert, wobei das Analysieren auf einer Messung der Anzahl von CTCs bezogen auf einen Schwellenwert von 5 zirkulierenden Tumorzellen pro 7,5 mL Blut basiert, wobei der Messwert oberhalb oder gleich diesem Schwellenwert auf eine niedrigere Überlebensprognose hinweist, wobei das Analysemittel aus der Gruppe bestehend aus Mitteln zur Kaplan-Meier-Analyse, Mitteln zur proportionalen Hazard-Regressionsanalyse nach Cox und Kombinationen davon ausgewählt ist.

9. Verfahren zum Abschätzen der Zeit zur Krankheitsprogression bei Patienten mit metastasierendem Brustkrebs zu irgendeinem Zeitpunkt während der Behandlung, umfassend:
a) Anreichern einer Fraktion einer Blutprobe mit einer gemischten Zellpopulation, die unter Verdacht steht zirkulierende Tumorzellen (circluating tumor cells, CTCs) zu enthalten, wobei die Fraktion die CTCs enthält;
b) Bestätigen, dass die strukturelle Integrität der CTCs intakt ist; und
c) Analysieren der intakten CTCs zu irgendeinem Zeitpunkt während der Nachbehandlung des Patienten, um die Zeit zur Krankheitsprogression zu bestimmten, wobei das Analysieren die Auszählung intakter CTCs des Patienten mit der Zeit zur Krankheitsprogression, die auf einem vorbestimmten statistischen Zusammenhang basiert, korreliert, wobei das Analysieren auf einer Messung der Anzahl von CTCs bezogen auf einen Schwellenwert von 5 zirkulierenden Tumorzellen pro 7,5 mL Blut basiert, wobei der Messwert oberhalb oder gleich diesem Schwellenwert auf eine kürzere Zeit zur Krankheitsprogression hinweist.

10. Verfahren nach Anspruch 9, wobei die zirkulierenden CTCs Ephithelzellen sind.

11. Verfahren nach Anspruch 9, wobei die Fraktion durch immunomagnetische Anreicherung erhalten wird, wobei die Probe mit magnetischen Teilchen, die an einen biospezifischen Liganden gebunden sind, der spezifisch an die CTCs bindet, gemischt wird, zum wesentlichen Ausschluss anderer Populationen und Aussetzen des Gemisches aus Probe und magnetischen Partikeln einem Magnetfeld, um eine Zellsuspension herzustellen, welche an CTCs angereichert ist, die an magnetische Partikel gebundenen sind.

12. Verfahren nach Anspruch 9, wobei der biospezifische Ligand ein gegen ein Oberflächenantigen von Epithelzellen gerichteter Antikörper ist.

13. Verfahren nach Anspruch 9, wobei die CTCs als das Oberflächenantigen von Epithelzellen EpCAM aufweisen.

14. Verfahren nach Anspruch 9, wobei die strukturelle Integrität durch ein Verfahren bestimmt wird, das aus einer Gruppe bestehend aus immunzytochemischen Verfahren, RT-PCR-Verfahren, PCR-Verfahren, FISH-Verfahren, Durchflusszytometrie-Verfahren, Bildzytometrie-Verfahren und Kombinationen davon ausgewählt ist.

15. Verfahren nach Anspruch 9, wobei das Analysieren auf einer Änderung bei der Auszählung der intakten CTCs basiert, die während des letzten Nachbeobachtungszeitraums auftritt, um auf die Zeit zur Krankheitsprogression hinzuweisen.

16. CTC-Analysesystem zum Abschätzen der Zeit zur Krankheitsprogression bei Patienten mit metastasierendem Brustkrebs zu irgendeinem Zeitpunkt während der Behandlung, wenn in dem Verfahren nach Anspruch 9 verwendet, wobei das CTC-Analysesystem umfasst:
a) Mittel zum Stabilisieren von Zellen in einer Blutprobe des Patienten, wobei das Mittel charakteristische Determinanten der CTCs in der Probe bewahrt, wobei das Stabilisierungsmittel aus der Gruppe bestehend aus anti-koagulierenden Mitteln, Stabilisatoren und Kombinationen davon ausgewählt ist;
b) Mittel zum Anreichern einer Fraktion der Probe, wobei die Fraktion intakte CTCs enthält, wobei das Anreicherungsmittel aus der Gruppe bestehend aus immunomagnetischen Mitteln, Dichtezentrifugation und Kombinationen davon ausgewählt ist;
c) Mittel zur Bestätigung der strukturellen Integrität der intakten CTCs, wobei das Bestätigungsmittel aus der Gruppe bestehend aus immunzytochemischen Mitteln, RT-PCR-Mitteln, PCR-Mitteln, FISH-Mitteln, Durchflusszytometrie-Mitteln, Bildzytometrie-Mittel und Kombinationen davon ausgewählt ist; und
d) Mittel zum Analysieren der intakten CTCs zu irgendeinem Zeitpunkt während der Nachbehandlung des Patienten, wobei das Mittel die Auszählung intakter CTCs mit der Zeit zur Krankheitsprogression, die auf einem vorbestimmten statistischen Zusammenhang basiert, korreliert, wobei das Analysieren auf einer Messung der Anzahl von CTCs bezogen auf einen Schwellenwert von 5 zirkulierenden Tumorzellen pro 7,5 mL Blut basiert, wobei der Messwert oberhalb oder gleich diesem Schwellenwert auf eine kürzere Zeit zur Krankheitsprogression hinweist, wobei das Analysemittel aus der Gruppe bestehend aus Mitteln zur Kaplan-Meier-Analyse, Mitteln zur proportionalen Hazard-Regressionsanalyse nach Cox und Kombinationen davon ausgewählt ist.

## Revendications

1. Procédé de pronostic de la survie globale chez des patients à tout point durant le traitement de patientes souffrant d'un cancer du sein métastasé comprenant :
a) l'enrichissement d'une fraction d'un échantillon de sang comprenant une population cellulaire mixte suspectée de contenir des cellules tumorales circulantes (CTC), ladite fraction contenant lesdites CTC ;
b) la confirmation du caractère intact de l'intégrité structurale desdites CTC ; et
c) l'analyse desdites CTC intactes à tout moment durant le traitement de suivi de la patiente pour déterminer la survie de la patiente, dans lequel ladite analyse corrèle la numération des CTC intactes de ladite patiente avec ledit pronostic de survie globale sur la base d'une association statistique prédéterminée, ladite analyse étant basée sur une mesure du nombre de CTC par rapport à un nombre seuil de 5 cellules tumorales circulantes pour 7,5 ml de sang, ladite mesure supérieure ou égale audit seuil indiquant que ledit pronostic de survie est inférieur.

2. Procédé selon la revendication 1, dans lequel lesdites CTC circulantes sont des cellules épithéliales.

3. Procédé selon la revendication 1, dans lequel ladite fraction est obtenue par enrichissement immunomagnétique, dans lequel ledit échantillon est mélangé avec des particules magnétiques couplées à un ligand biospécifique qui se lie spécifiquement auxdites CTC à l'exclusion sensible d'autres populations et la soumission du mélange échantillon-particules magnétiques à un champ magnétique pour produire une suspension cellulaire enrichie en CTC liées aux particules magnétiques.

4. Procédé selon la revendication 1, dans lequel ledit ligand biospécifique est un anticorps dirigé contre un antigène de la surface des cellules épithéliales.

5. Procédé selon la revendication 4, dans lequel lesdites CTC comprennent EpCAM en tant que ledit antigène de la surface des cellules épithéliales.

6. Procédé selon la revendication 1, dans lequel ladite intégrité structurale est déterminée par une procédure choisie dans un groupe constitué des procédures immunocytochimiques, des procédures de RT-PCR, des procédures de PCR, des procédures de FISH, des procédures de cytométrie en flux, des procédures de cytométrie en images, et de leurs combinaisons.

7. Procédé selon la revendication 1, dans lequel ladite analyse est basée sur un changement de ladite numération des CTC intactes survenant durant la dernière période de suivi pour indiquer ledit pronostic de survie.

8. Système d'analyse des CTC pour l'estimation de la survie globale chez des patientes souffrant d'un cancer du sein métastasé à tout point durant le traitement, lors d'une utilisation dans le procédé selon la revendication 1, comprenant :
a) un moyen de stabilisation des cellules dans un échantillon de sang provenant de ladite patiente, ledit moyen préservant les déterminants caractéristiques des CTC dans ledit échantillon, dans lequel ledit moyen de stabilisation est choisi dans le groupe constitué d'agents anticoagulants, d'agents stabilisants, et de leurs combinaisons ;
b) un moyen d'enrichissement d'une fraction dudit échantillon, ladite fraction contenant des CTC intactes, dans lequel ledit moyen d'enrichissement est choisi dans le groupe constitué d'un moyen immunomagnétique, de centrifugation en gradient de densité, et de leurs combinaisons ;
c) un moyen de confirmation de l'intégrité structurale desdites CTC intactes, dans lequel ledit moyen de confirmation est choisi dans le groupe constitué des moyens immunocytochimiques, des moyens de RT-PCR, des moyens de PCR, des moyens de FISH, des moyens de cytométrie en flux, des moyens de cytométrie en images, et de leurs combinaisons ; et
d) un moyen d'analyse desdites CTC intactes à tout moment durant le traitement de suivi de la patiente, dans lequel ledit moyen corrèle ladite numération des CTC intactes avec ladite survie globale sur la base d'une association statistique prédéterminée, ladite analyse étant basée sur une mesure du nombre de CTC par rapport à un nombre seuil de 5 cellules tumorales circulantes pour 7,5 ml de sang, ladite mesure supérieure ou égale audit seuil indiquant que ledit pronostic de survie est inférieur, dans lequel ledit moyen d'analyse est choisi dans le groupe constitué des moyens d'analyse de Kaplan-Meier, des moyens d'analyse de la régression à risques proportionnels de Cox, et de leurs combinaisons.

9. Procédé d'estimation du temps pour la progression de la maladie chez des patientes souffrant d'un cancer du sein métastasé à tout point de temps durant le traitement comprenant :
a) l'enrichissement d'une fraction d'un échantillon de sang comprenant une population cellulaire mixte suspectée de contenir des CTC, ladite fraction contenant lesdites CTC ;
b) la confirmation du caractère intact de l'intégrité structurale desdites CTC ; et
c) l'analyse desdites CTC intactes à tout moment durant le traitement de suivi de la patiente pour déterminer le temps pour la progression de la maladie, dans lequel ladite analyse corrèle la numération des CTC intactes de ladite patiente avec ledit temps pour la progression de la maladie sur la base d'une association statistique prédéterminée, ladite analyse étant basée sur une mesure du nombre de CTC par rapport à un nombre seuil de 5 cellules tumorales circulantes pour 7,5 ml de sang, ladite mesure supérieure ou égale audit seuil indiquant que ledit temps pour la progression de la maladie est inférieur.

10. Procédé selon la revendication 9, dans lequel lesdites CTC circulantes sont des cellules épithéliales.

11. Procédé selon la revendication 9, dans lequel ladite fraction est obtenue par enrichissement immunomagnétique, dans lequel ledit échantillon est mélangé avec des particules magnétiques couplées à un ligand biospécifique qui se lie spécifiquement auxdites CTC à l'exclusion sensible d'autres populations et la soumission du mélange échantillon-particules magnétiques à un champ magnétique pour produire une suspension cellulaire enrichie en CTC liées aux particules magnétiques.

12. Procédé selon la revendication 9, dans lequel ledit ligand biospécifique est un anticorps dirigé contre un antigène de la surface des cellules épithéliales.

13. Procédé selon la revendication 9, dans lequel lesdites CTC comprennent EpCAM en tant que ledit antigène de la surface des cellules épithéliales.

14. Procédé selon la revendication 9, dans lequel ladite intégrité structurale est déterminée par une procédure choisie dans un groupe constitué des procédures immunocytochimiques, des procédures de RT-PCR, des procédures de PCR, des procédures de FISH, des procédures de cytométrie en flux, des procédures de cytométrie en images, et de leurs combinaisons.

15. Procédé selon la revendication 9, dans lequel ladite analyse est basée sur un changement de ladite numération des CTC intactes durant la dernière période de suivi pour indiquer ledit temps pour la progression de la maladie.

16. Système d'analyse des CTC pour estimer le temps pour la progression de la maladie chez une patiente souffrant d'un cancer du sein métastasé à tout moment durant le traitement, lors d'une utilisation dans le procédé selon la revendication 9, ledit système d'analyse des CTC comprenant :
a) un moyen de stabilisation des cellules dans un échantillon de sang provenant de ladite patiente, ledit moyen préservant les déterminants caractéristiques des CTC dans ledit échantillon, dans lequel ledit moyen de stabilisation est choisi dans le groupe constitué d'agents anticoagulants, d'agents stabilisants, et de leurs combinaisons ;
b) un moyen d'enrichissement d'une fraction dudit échantillon, ladite fraction contenant des CTC intactes, dans lequel ledit moyen d'enrichissement est choisi dans le groupe constitué d'un moyen immunomagnétique, de centrifugation en gradient de densité, et de leurs combinaisons ;
c) un moyen de confirmation de l'intégrité structurale desdites CTC intactes, dans lequel ledit moyen de confirmation est choisi dans le groupe constitué des moyens immunocytochimiques, des moyens de RT-PCR, des moyens de PCR, des moyens de FISH, des moyens de cytométrie en flux, des moyens de cytométrie en images, et de leurs combinaisons ; et
d) un moyen d'analyse desdites CTC intactes à tout moment durant le traitement de suivi de la patiente, dans lequel ledit moyen corrèle ladite numération des CTC intactes avec ledit temps pour la progression de la maladie sur la base d'une association statistique prédéterminée, ladite analyse étant basée sur une mesure du nombre de CTC par rapport à un nombre seuil de 5 cellules tumorales circulantes pour 7,5 ml de sang, ladite mesure supérieure ou égale audit seuil indiquant que ledit temps pour la progression de la maladie est inférieur, dans lequel ledit moyen d'analyse est choisi dans le groupe constitué des moyens d'analyse de Kaplan-Meier, des moyens d'analyse de la régression à risques proportionnels de Cox, et de leurs combinaisons.
